# EUROPÄISCHE PATENTSCHRIFT

(11) **EP 0 931 038 B1**
(45) Veröffentlichungstag und Bekanntmachung des Hinweises auf die Patenterteilung: **16.04.2003**
(21) Anmeldenummer: 97923042.2
(22) Anmeldetag: 09.05.1997
(51) Int. Cl.: C07B 61/00, C07K 1/04, C07D 473/34

(54) **NEUE SUBSTANZBIBLIOTHEK UND DAMIT HERGESTELLTE SUPRAMOLEKULARE KOMPLEXE**
NOVEL SUBSTANCE LIBRARY AND SUPRAMOLECULAR COMPLEXES PRODUCED THEREWITH
NOUVELLE BIBLIOTHEQUE DE SUBSTANCES ET COMPLEXES SUPRAMOLECULAIRES OBTENUS AU MOYEN DE LADITE BIBLIOTHEQUE

(30) Priorität: 14.05.1996 DE 19619373
(43) Veröffentlichungstag der Anmeldung: 28.07.1999
(73) Patentinhaber: Nanogen Recognomics GmbH, 65926 Frankfurt am Main (DE)
(72) Erfinder: MICULKA, Christian, D-65929 Frankfurt am Main (DE); WINDHAB, Norbert, D-65795 Hattersheim (DE); QUINKERT, Gerhard, D-61479 Glashütten (DE); ESCHENMOSER, Albert, CH-8700 Küsnacht (CH)
(74) Vertreter: Ackermann, Joachim, Dr.
(86) Internationale Anmeldenummer: EP9702387
(87) Internationale Veröffentlichungsnummer: WO97043232

(56) Entgegenhaltungen:
- WO-A-95/19567
- WO-A-97/00267
- DE-A- 4 343 591
- JOURNAL OF THE AMERICAN CHEMICAL SOCIETY, Bd. 118, Nr. 7, 21.Februar 1996, DC US, Seiten 1813-1814, XP002040585 Y CHENG ET AL: "Sequence-selective peptide binding with a peptido-A,B-trans-steroidal receptor selected from an encoded combinatorial receptor library" in der Anmeldung erwähnt

## Beschreibung

Die vorliegende Erfindung betrifft eine Substanzbibliothek, ein Verfahren zu deren Herstellung, ein Verfahren zur Herstellung von supramolekularen Komplexen unter Verwendung dieser Substanzbibliothek und die Verwendung der mittels der Substanzbibliothek hergestellten supramolekularen Komplexe sowie die Verwendung der Substanzbibliothek selbst.

Kombinatorische Strategien sind wichtige Ansätze in der Suche nach neuen Wirkstoffen, besonders im Hinblick auf die Auffindung von Leitstrukturen sowie deren Optimierung: Man synthetisiert Ensembles strukturell verwandter Verbindungen simultan und meist automatisiert; die hierbei anfallenden Gemische (sog. Bibliotheken) enthalten Hunderte, Tausende oder gar Millionen von Einzelverbindungen in jeweils geringer Menge. Wird im Gemisch die Wirksamkeit einer Komponente durch Screening nachgewiesen, so beschränkt sich die weitere Tätigkeit des Chemikers auf die Bestimmung der Identität, da das Syntheseprotokoll ja bekannt ist.

Waren es anfangs vornehmlich Substanzbibliotheken linear konstituierter Moleküle wie Peptide [K.S. Lam, S.E. Salmon, E.M. Hersh, V.J. Hruby, W.M. Kazmierski, R.J. Knapp, Nature 1991, 354, 82-84], so sind es neuerdings die vor allem im Wirkstoffbereich wichtigen "kleinen" Moleküle wie Heterocyclen [L.A. Thompson, J.A. Ellman, Chem. Rev. 1996, 96, 555-600], die im Mittelpunkt des Interesses stehen. Ziel ist die Erzeugung von molekularer Diversität, um die Auffindung von Leitstrukturen bzw. deren Optimierung zu beschleunigen.

Das Charakteristikum der herkömmlichen kombinatorischen Chemie ist, daß die Synthese unter kinetischer Kontrolle abläuft und daß die Variation durch Synthese von der Selektion getrennt ist. Dies betrifft die in vitro-Evolution von RNA-Aptameren [J.R. Lorsch, J.W. Szostak, Nature 1994, 371, 31-36.] ebenso wie die Rezeptorsuche mit kombinatorischen Methoden [Y. Cheng, T. Suenaga, W.C. Still, J. Am. Chem. Soc. 1996, 118, 1813-1814], dabei wurden zwei kurze Peptidbibliotheken an einem Steroidgerüst aufgebaut und damit irreversibel verknüpft.

Aufgabe der vorliegenden Erfindung ist es, durch Bereitstellung einer neuen Art von Substanzbibliothek die Zahl der Bindungsstellen für die mittels einer Substanzbibliothek auf deren Bindungseigenschaften untersuchten Liganden bzw. Substratmoleküle durch reversible Kombination jeweils zweier oder mehrerer gleicher oder verschiedener in der Substanzbibliothek enthaltenen molekularen Spezies gegenüber herkömmlichen Substanzbibliotheken um Größenordnungen zu erhöhen, wobei die Kombination der in der Substanzbibliothek enthaltenen molekularen Spezies erst in Gegenwart des zu untersuchenden Substratmoleküls über die entsprechenden bindenden Wechselwirkungen mit den molekularen Spezies erfolgen soll.

Eine weitere Aufgabe der vorliegenden Erfindung ist es, supramolekulare Komplexe bereitzustellen, welche durch die Kombination der in der Substanzbibliothek enthaltenen molekularen Spezies und durch die bindenden Wechselwirkungen mit dem zu untersuchenden Substratmolekül entstehen.

Derartige Substanzbibliotheken und solche supramolekularen Komplexe sollten sich zur Herstellung von Arzneiwirkstoffen, Pflanzenschutzwirkstoffen, Katalysatoren oder zur Diagnose von Krankheiten eignen.

Die eingangs gestellte Aufgabe wird gelöst durch eine Substanzbibliothek, erhältlich durch Koppelung von verschiedenen oder gleichen molekularen Spezies, welche vorzugsweise in einer Substanzbibliothek enthalten sind, an ein molekulares Paarungssystem. Gegenstand der Erfindung sind folglich Substanzbibliotheken, enthaltend ein molekulares Paarungssystem, wobei mindestens zwei der molekularen Spezies, die das Paarungssystem bilden, jeweils an mindestens eine gleiche oder verschiedene molekulare Spezies kovalent gebunden sind und wobei das molekulare Paarungssystem reversibel durch nicht-kovalente Kräfte ausgebildet wird.

Mittels der Substanzbibliothek gemäß der vorliegenden Erfindung lassen sich supramolekulare Komplexe dadurch herstellen, daß man die Substanzbibliothek einer Wechselwirkung mit einem Substrat aussetzt und den dabei gebildeten supramolekularen Komplex identifiziert und gegebenenfalls isoliert.

Die vorliegende Erfindung betrifft demgemäß auch die Bereitstellung eines so hergestellten supramolekularen Komplexes, der sich beispielsweise zur Herstellung von Arzneistoffen, Pflanzenschutzwirkstoffen, Katalysatoren, zur Diagnose von Krankheiten sowie der Herstellung von entsprechenden Diagnose-Kits eignet,

in gleicher Weise ist auch die Vorstufe dieser supramolekularen Komplexes, nämlich die erfindungsgemäße Substanzbibliothek zur Herstellung von Arzneistoffen, Pflanzenschutzwirkstoffen, Katalysatoren und zur Diagnose von Krankheiten einschließlich zur Herstellung der entsprechenden Diagnose-Kits geeignet.

Im folgenden werden die vorgenannten oder nachfolgend zur Erläuterung der Erfindung sowie in den Patentansprüchen verwendeten allgemeinen Begriffe definiert.

Molekulare Spezies: Beispielsweise linearkonstituierte Moleküle wie Peptide, insbesondere Proteine, Peptoide, lineare Oligo- oder Polysaccharide. Nukleinsäuren und deren Analoga, oder beispielsweise Monomere wie Heterocyclen insbesondere Stickstoffheterocyclen, oder nichtlinearkonstituierte Moleküle wie verzweigte Oligo- oder Polysaccharide oder auch Antikörper.

Supramolekularer Komplex: Entsteht durch Assoziation von zwei oder mehreren molekularen Spezies, die durch nicht-kovalente Kräfte zusammengehalten werden.

Paarungssysteme: Supramolekulare Systeme nicht-kovalenter Wechselwirkungen, die durch Selektivität, Stabilität und Reversibilität gekennzeichnet sind und deren Eigenschaften bevorzugt thermodynamisch, wie z. B. durch Temperatur, pH-Wert, Konzentration, beeinflußt werden. Beispiele sind bevorzugt Pyranosyl-RNA, CNA, DNA, RNA, PNA.

Wechselwirkungen sind bevorzugt Wasserstoffbrücken, Salzbrücken, Stapelung ("Stacking"), Metalligandierungen, Charge-Transfer-Komplexe und hydrophobe Wechselwirkungen.

Substanzbibliothek: Ensemble von sich strukturell unterscheidenden Verbindungen, bevorzugt oligomere oder polymere Peptide, Peptoide, Saccharide, Nucteinsäuren, Ester, Acetale oder Monomere wie Heterocyclen, Lipide, Steroide.

Substrat: Moleküle, bevorzugt Arzneistoffe und Pflanzenschutzwirkstoffe, Metaboliten, physiologische Botenstoffe, Derivate von Leitstrukturen, Substanzen, die im menschlichen oder tierischen Körper im Fall von krankhaften Veränderungen produziert oder im erhöhten Maß produziert werden, Übergangszustandanaloga oder auch Peptide, insbesondere Proteine, Peptoide, lineare Oligo- oder Polysaccharide, Nukleinsäuren und deren Analoga, oder beispielsweise Monomere wie Heterocyclen, insbesondere Stickstoffheterocyclen, oder nichtlinearkonstituierte Moleküle wie verzweigte Oligo- oder Polysaccharide oder auch Antikörper sowie Substanzbibliotheken, zudem Angriffspunkte von Pharmaka, vorzugsweise Rezeptoren, spannungsabhängige Ionenkanäle,Transporter, Enzyme und Biosynthese-Einheiten von Mikroorganismen.

Übergangszustandanaloga: Molekulare synthetische Spezies, die dem anzunehmenden Übergangszustand einer chemischen Reaktion strukturell ähnlich, aber im Gegensatz dazu stabil sind.

Identifizierung kann Isolierung oder Charakterisierung des supramolekularen Komplexes beinhalten, bevorzugt aber Unterscheidung anhand der besonderen Eigenschaften des supramolekularen Komplexes aus an Paarungssystemen gekoppelten Substanzbibliotheken und Substrat, bevorzugt unterschiedliches chromatographisches, elektrophoretisches, spektroskopisches oder Signal-(Markierungs-) Verhalten im Vergleich zu nicht-komplexierten Spezies oder durch kovalente (chemische) Fixierung der an der Komplexbildung beteiligten Spezies.

CNA: Cyclohexylnucleooligo-Amid; stellt eine synthetische Variante der DNA-Struktur dar, bei welcher das Phosphat-Zucker-Rückgrat durch 2-(3-Aminocyclohexyl)-ethansäureeinheiten ersetzt ist, wobei die Einheiten peptidartig miteinander verknüpft sind und die 3-Amino-Cyclohexylsubstituenten in Position 4 jeweils mit einer Nukleobase versehen sind.

Vorzugsweise zeichnet sich die Substanzbibliothek gemäß der vorliegenden Erfindung dadurch aus, daß das Paarungssystem aus einem längeren und zwei kürzeren Basensträngen besteht, wobei die beiden kürzeren Stränge an unterschiedlichen Stellen komplementär zum längeren Strang, jedoch zueinander nicht komplementär sind und wobei im Falle der Basenpaarung mit dem längeren Strang zwischen den kürzen Strängen eine Lücke von wenigstens einer Base verbleibt, während im Bereich dieser Lücke entsprechend deren Größe auf dem längeren Strang mindestens eine Base ungepaart bleibt, wobei jeweils diejenigen Basen der beiden kürzeren Stränge, die sich am Anfang bzw. am Ende der Paarungslücke befinden, über einen Linker mit jeweils einer molekularen Spezies verknüpft sind, während mindestens eine der ungepaarten Basen des längeren Stranges mit einer molekularen Spezies über einen Linker verknüpft ist.

Peptide unterschiedlicher Eigenschaften können beispielsweise durch Verknüpfung mit paarenden Oligonucleotid-Enden kontrolliert zu Zweier- oder Dreiergruppen reversibel zusammentreten. Damit werden im Experiment durch die Vielzahl von Kombinationsmöglichkeiten um Größenordnungen mehr verschiedene Bindungsstellen erzeugt, als Peptide synthetisiert wurden.

Die Verwirklichung des Prinzips der kombinatorischen Variation und Selektion unter thermodynamischer Kontrolle sowie deren Verknüpfung stellt einen elementaren Technologiesprung in der kombinatorischen Methodik dar: Erst in Gegenwart des Substrats bildet sich durch Kombination der zugehörige Rezeptor.

Dieser Rezeptor reagiert somit auf die Gegenwart des Substrats: Setzt man dieses einem Antigen gleich, so kann das vorliegende System in Analogie als "künstliches Immunsystem" betrachtet werden.

Die Natur hat eine bemerkenswerte Anzahl von Moleküle hervorgebracht, welche die komplexen Prozesse der lebenden Organismen ausführen - von der Immunantwort und Katalyse bis zur Signalübertragung. Dabei greift sie auf eine breite kombinatorische Bibliothek von Vorläufermolekülen zurück und überprüft diese auf die gewünschten Eigenschaften. Das wahrscheinlich bedeutendste Beispiel für diese Strategie stellt das Immunsystem dar, welches eine enorme molekulare Diversität erzeugen kann und diese auf hochaffine und selektive Rezeptoren für fremde Antigene durchsucht. Auch das Zusammentreten an sich nicht oder nur schwach bindender Moleküle zu einem stabilen Bindungskomplex ist ein in der Natur verbreitetes Prinzip (Heteromere [D.E. Clapham, Nature 1996, 379, 297-299]) dessen Bedeutung für die Anwendung in der kombinatorischen Chemie noch nicht erkannt wurde.

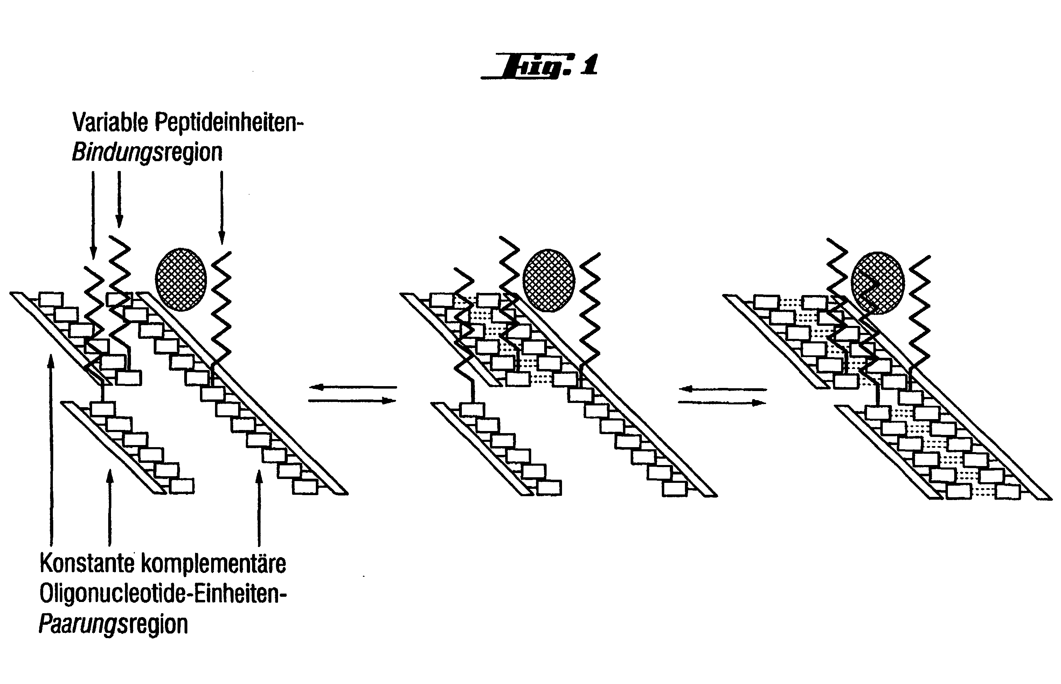

Fig 1. zeigt schematisiert den Aufbau und den Ablauf der Bildung eines solchen Rezeptors: An einem beispielsweise aus 13 Monomerbausteinen aufgebauten Oligonucleotid wird über eine Linkereinheit eine kurze Peptidkette (als Bibliothek) am Mittelbaustein kovalent angebunden. Analog wird an den beiden Endeinheiten der kurzen Oligonucleotide aus 6 Monomereinheiten vorgegangen.

Wird nun diesen drei Einheiten das Substrat (Ellipse) angeboten, so setzt ein Wettbewerb um die beste Bindung der Peptidteile an das Substrat ein: Die Paarung zwischen den Oligonucleotiden sorgt für räumliche Annäherung der peptidischen Teile. Entscheidend ist die Reversibilität der Einzelschritte, wodurch die einzelnen Peptidbereiche solange ausgetauscht werden, bis der stabilste Komplex gefunden wurde. Dieser unter thermodynamischer Kontrolle ablaufende Vorgang entspricht einem selbsttätigen experimentellen "Molecular Modelling". In einem solchen Experiment wird tatsächlich die Gesamtheit der möglichen transient auftretenden Kombinationen der drei Bibliotheken der Selektion unterworfen. Dieser Austauschvorgang ist temperaturabhängig, d. h. bei höherer Temperatur werden die einzelnen Stränge öfter ausgewechselt, zugleich werden aber auch die Wechselwirkungen der Peptidteile mit dem Substrat schwächer.

Nach Einfrieren des Gleichgewichts, kovalentes Cross-Linking der Paarungspartner, Isolierung und Dekomplexierung wird der Rezeptor in freier Form erhalten.

Es wurde daher ein Verfahren zur Herstellung supramolekularer Komplexe ausgearbeitet, dadurch gekennzeichnet, daß man Verbindungsbibliotheken an Paarungssysteme koppelt.

Die mit den nachfolgenden Verfahren unter thermodynamischer Kontrolle hergestellten und gekoppelt unter thermodynamischer Kontrolle selektionierten supramolekularen Komplexe kommen dann zum Einsatz, wenn Moleküle oder Molekülbereiche erkannt werden sollen. Der Vorteil liegt darin, daß die immer gleichen Bibliotheken in Kombination immer neue Selektionsprobleme sehr schnell lösen können.

Diese sind vor allem:
a) Molekulare Erkennung biologisch relevanter Substanzen, d. h. in-vitro Diagnostik. Gerade die Entwicklung diagnostischer Methoden muß mit der Vielfalt der zu erkennenden Substrate, wie Metaboliten oder z. B. sich ständig mutierenden Erregern, Schritt halten, sodaß der Nutzen dieses Verfahrens offensichtlich wird.
b) Molekulare Erkennung biologisch relevanter Substanzen, d. h. Drug Design. Das beschriebene Verfahren erzeugt hochselektive supramolekulare Komplexe, die selbst als Wirkstoffe oder als Modelle für die Wirkstoffentwicklung dienen, indem sie zum Beispiel an pharmakologische Rezeptoren binden und damit stimulieren oder blockieren. Auf der anderen Seite dienen die supramolekularen Komplexe als Rezeptoren in der Wirkstoffentwicklung, da mit ihrer Hilfe ein Wechselwirkungsprofil der Wirkstoffe erstellt werden kann.
c) Thermodynamisch kontrollierte Konstitution von katalytisch aktiven supramolekularen Komplexen z. B. dadurch, daß man im Sinne der Verwendung als katalytischer Antikörper [L.C. Hsieh-Wilson, X.-D. Xiang, P.G. Schultz, Acc. Chem. Res. 1996, 29, 164-170] Übergangszustandanaloga als Substrate anbietet.

### Durchführungsbeispiel 1

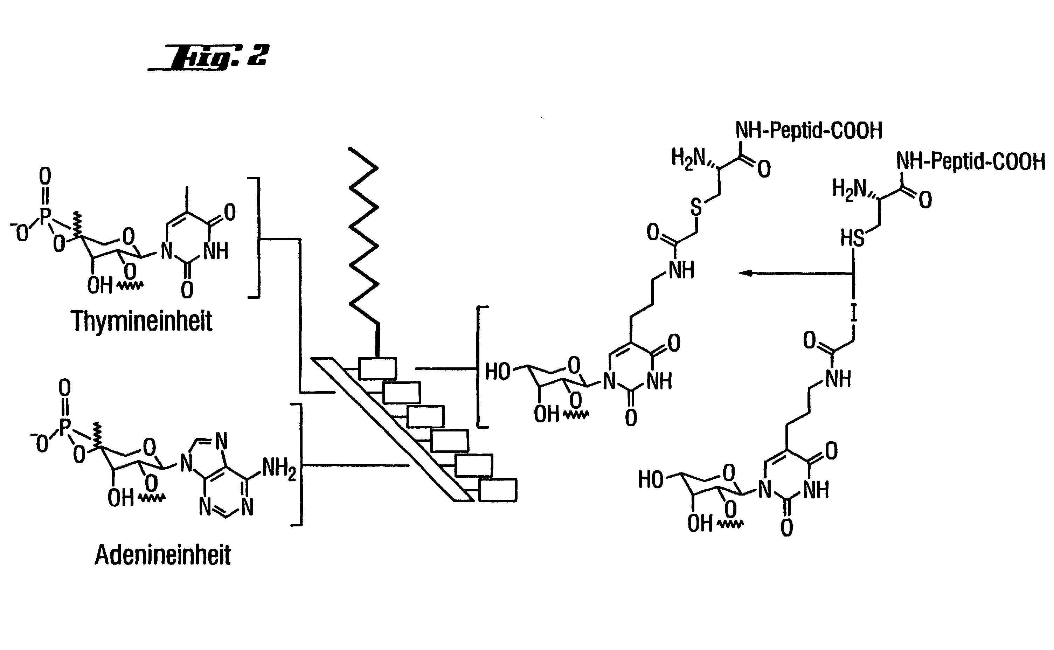

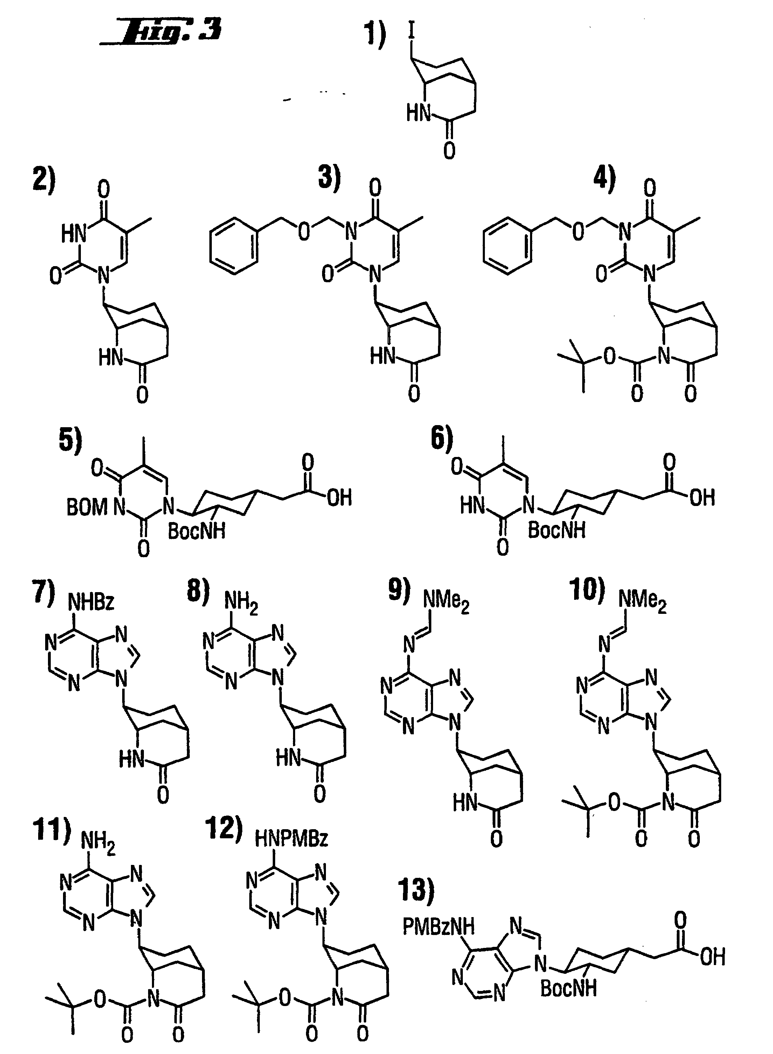

Als Paarungssystem wird die Pyranosyl-RNA verwendet (s. Fig. 2), deren Herstellung und Eigenschaften wohlbekannt sind [S. Pitsch, S. Wendeborn, B. Jaun, A. Eschenmoser, Helv. Chim. Acta 1993, 76, 2161-2183]. Ausgehend von D-Ribose und den Nucleobasen Adenin und Thymin werden wie darin beschrieben die kopplungsfähigen Phosphoramidite hergestellt und mit einem Oligonucleotidsynthesizer die gewünschten Hexamer- bzw. Tridecamer-Sequenzen hergestellt. Das Tridecamer hat die Sequenz 2'-AATTAAT*ATATAT, ein Hexamer hat die Sequenz 2'-T*TAATT-4', das andere Hexamer hat die Sequenz 2'-ATATAT*-4', wobei T* den Linker-Nucleotidbaustein darstellt. Der Linker-Nucleotidbaustein wird nach literaturbekannten Methoden ausgehend vom Uracil-Nucleosid synthetisiert: Jodierung [W.-W. Sy, Synth. Comun. 1990, 20, 3391-3394]. Umsetzung mit Propargylphthalimid, Hydrierung [K.J. Gibson, S.J. Benkovic, Nucteic Acids Res. 1987, 15, 6455-6467] liefert den gewünschten Baustein. Hydrazinolyse und Jodacetylierung des Oligonucleotides erfolgt wie in der Literatur beschrieben [T. Zhu, S. Stein, Bioconjugate Chem. 1994, 5, 312-315]. Als Verbindungsbibliotheken werden Tetrapeptide ausgehend von kommerziell erhältlichen Aminosäuremonomeren mit einem multiplen Peptidsynthesizer hergestellt, wobei N-terminal als Linker-Einheit ein Cysteinrest vorgesehen wird. Man teilt die Bibliothek in drei Portionen und läßt in wäßriger, gepufferter Lösung bei Raumtemperatur jeweils mit den zwei Hexamer-Sequenzen bzw. der Tridecamer-Sequenz zu den gewünschten Konjugaten reagieren, die mittels Reverse Phase-Chromatographie gereinigt werden [T. Zhu, S. Stein, Bioconjugate Chem. 1994, 5, 312-3151. Die Paarung der komplementären Einheiten wird anhand der Abnahme der UV-Extinktion im Paarungsexperiment nachgewiesen.

### Durchführungsbeispiel 2

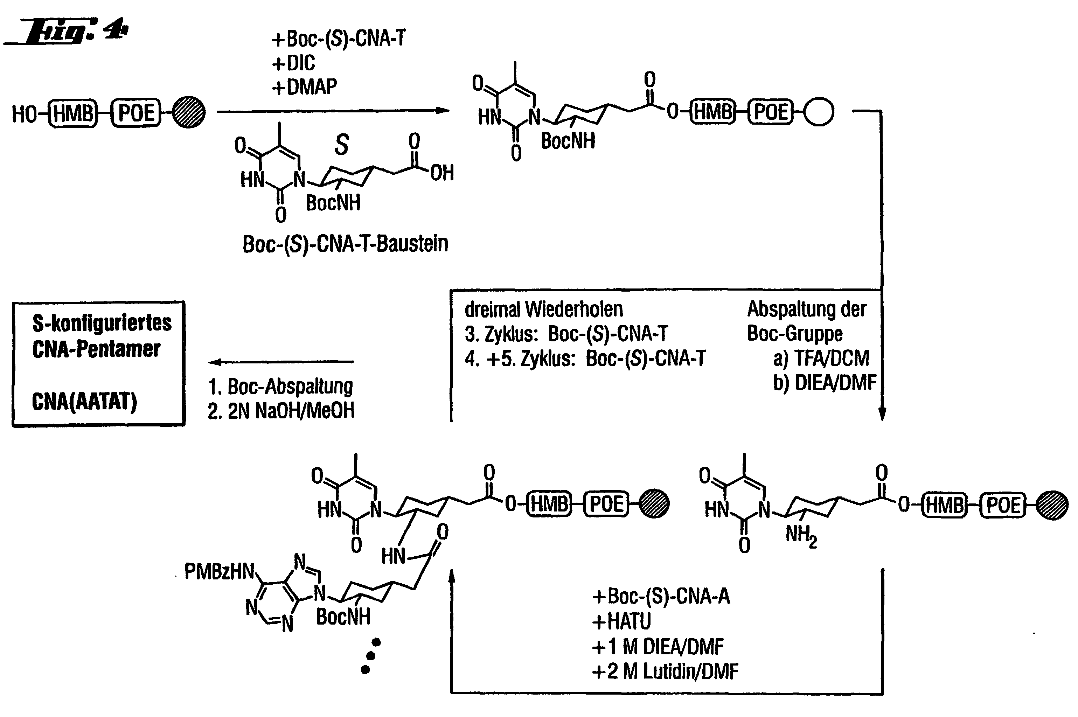

### Festphasensynthese eines CNA-Pentamers (Fig. 4)

Die Synthese des CNA-Oligomers erfolgte analog zur Peptid- oder Oligonucleotidsynthese, durch schrittweisen Einbau einzelner Bausteine an fester Phase. Dabei wurden die notwendigen Reagenzien im Überschuß zugesetzt und nicht umgesetzt Mengen durch einfache Waschschritte wieder entfernt. Als polymerer Träger wurde ein Polyoxyethylen-(POE)-Polystyrol-Copolymer (Tentagel S HMB, 0,23 mmol/g) verwendet, das sowohl in wäßriger Lösung als auch in organischen Lösungsmitteln gute Quelleigenschaften besitzt.

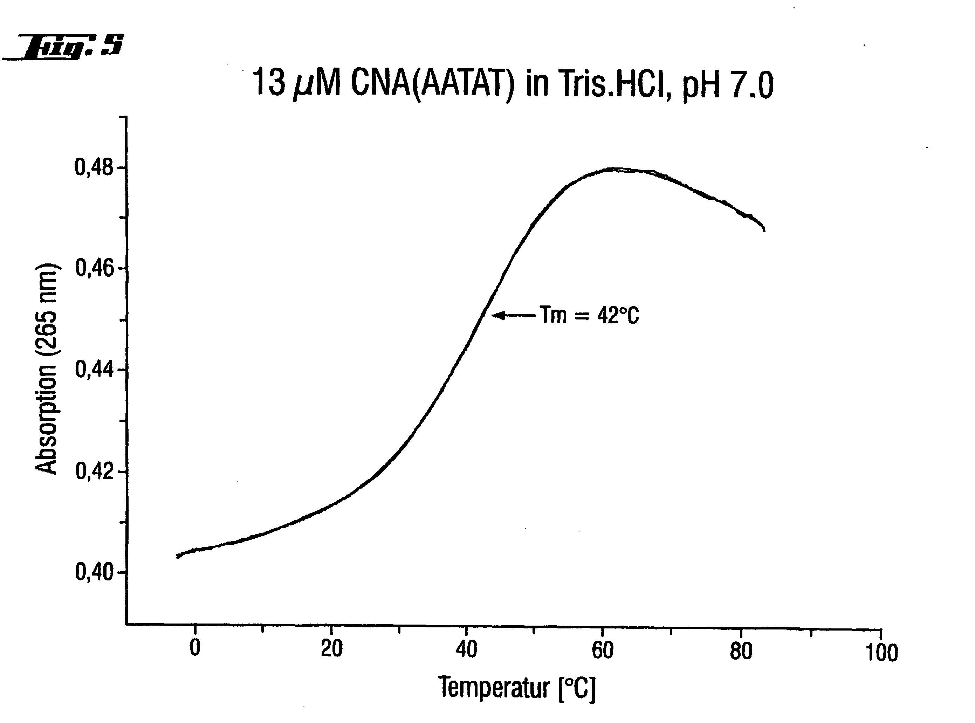

Die Aminoethylfunktionen des Polymers waren mit einem Hydroxymethylbenzoyl-(HMB)-Linker derivatisiert; die Beladung mit dem ersten Baustein erfolgte unter Verwendung eines 5-fachen Überschusses nach der Methode des symmetrischen Anhydrids (Zugabe von 2,5 eq DIC) und durch Zusatz des Acylierungskatalysators DMAP (2,5 eq) innerhalb von 20 h in DCM. Die erhaltene Beladung betrug 0,17 mmol/g. Die Boc-Schutzgruppe der Aminofunktion wurde mit 50 % TFA in DCM abgespalten, anschließend wurde das Harz mit 1 M DIEA/DMF neutralisiert. Die nachfolgenden Zyklen bestanden aus repetitiver Kupplung des nächsten Monomeren und Abspaltung der Boc-Schutzgruppe. Die Kupplungen erfolgten nach Voraktivierung des Monomer-Bausteins (3 eq.) mit dem Aktivierungsreagenz HATU (3 eq.) in DMF (40 µl) und unter Zusatz von 1 M DIEA/DMF (6 eq.) und 2 M Lutidin/DMF (12 eq.). Die Kupplungszeiten betrugen 3-4 h bei Raumtemperatur. Nach vier Kupplungszyklen wurden die N-terminale Boc-Schutzgruppe abgespalten und das Pentamer mit 2 N NaOH in Methanol innerhalb von 15 min vom Harz gespalten. Die Abspaltungslösung wurde vom Harz abfiltriert und 2 Std. bei 55°C gehalten. Nach Neutralisation mit 2 N HCI erfolgte die Aufreinigung mit C18-RP-HPLC (Hibar Fertigsäule 250-4, RP-18,5 µm) mit Gradientenelution (1 ml/min) von10 % auf 40 % B in 30 min (Lösungsmittel A: Wasser + 0,1 % TFA, B: Acetonitril + 0,1 % TFA).
Die Synthese von CNA(AATAT) wurde mit 10 mg (1,7 µmol) Tentagel-HMB-Harz durchgeführt, das mit (S)-CNA-Thymin-Monomerbaustein beladen war. Es handelte sich ausschließlich um CNA-Bausteine mit S-Konfiguration. Die Abfolge der Sequenz von links nach rechts entspricht der in der Peptidchemie üblichen Schreibweise von N- zum C-Terminus.
CNA (AATAT): HPLC : Rt = 14,30 min; UV: λmax = 264 nm; ESI-MS:
[M+H]⁺_{calc} 1362,0, [M+2H]²⁺_{calc} 681,0; [M+H]⁺ₑₓₚ 1361,8, [M+2H]²⁺ₑₓₚ 681,5.
Nach Entsalzung des CNA-Pentamers CNA (AATAT) wurden auf einem Perkin Elmer Lambda 2 UV-VIS Spektrometer die temperaturabhängigen Extinktionen bei 265 nm bei sechs verschiedenen Konzentrationen über einen Bereich von 0 bis 80°C gemessen (1,5-50 µM in TrisHCl Puffer bei pH 7,0). Die erste Ableitung dieser reversiblen, sigmoiden Umwandlungskurven ergibt die Schmelztemperatur (Tm = 42°C bei 13 µM) (Fig. 5).

### - Festphasensynthese eines Peptid-CNA-Konjugates

Das oben beschriebene CNA-Pentamer wurde vor der Abspaltung vom Harz um eine Dipeptidbibliothek am N-Terminus verlängert. Die Sequenz lautet XO-CNA(AATAT). X stellt eine Mischposition dar, in der die fünf L-Aminosäuren Alanin, Asparaginsäure, Leucin, Lysin und Serin variiert werden. O stellt eine definierte Position dar, wobei für diese Subbibliothek O = L-Lysin gewählt wurde. Die Kupplung von Boc-Lys(Fmoc)-OH an das Boc-entschützte CNA-Pentamer CNA(AATAT) erfolgte nach Voraktivierung des Aminosäure-Bausteins (6 eq.) mit dem Aktivierungsreagenz HATU (6 eq.) in DMF und unter Zusatz von 1 M DIEA/DMF (7 eq.). Die Kupplungszeit betrug 3 h. Die Einführung der X-Position erfolgte nach der Split-Resin-Methode. Nach Abspaltung der N-terminalen Boc-Schutzgruppe wurde die Harzmenge (5 mg) mit 100 µl DMF:DCM (1:1) versetzt und in fünf gleichgroße Portionen zu je 20 µl aufgeteilt. Die Kupplung der einzelnen Aminosäuren erfolgte parallel in separaten Reaktionsgefäßen mit je ca. 1 mg Oligomerharz. Die einzelnen Bocgeschützten Aminosäuren, Boc-Ala-OH, Boc-Asp(OFm)-OH, Boc-Leu-OH, Boc-Ser-OH und Boc-Lys(Fmoc)-OH wurden in 50-fachem Überschuß nach Voraktivierung mit HATU (50 eq.) und unter Zugabe von 1 M DIEA/DMF (100 eq.) 3 h bei Raumtemperatur gekuppelt. Nach Abspaltung der N-terminalen Boc-Schutzgruppen wurde die Fmoc-Schutzgruppen mit 40 % Piperidin/DMF (20 min) entfernt. Die Peptid-CNA-Oligomerkonjugate wurden jeweils mit 2 N NaOH in Methanol innerhalb von 15 min vom Harz gespalten. Die Abspaltungslösung wurde vom Harz abfiltriert und 2 Std. bei 55°C gehalten. Nach Neutralisation mit 2 N HCI erfolgte die Aufreinigung mit C18-RP-HPLC (Hibar Fertigsäure 250-4, RP-18, 5 µm) mit Gradientenelution (1 ml/min) von 10 % B auf 40 % B in 30 min (Lösungsmittel A: Wasser + 0,1 % TFA, B: Acetonitril + 0,1 % TFA).

| | |
|---|---|
| HPLC: | Ala-Lys-CNA(AATAT)Rt = 15,47 min Asp-Lys-CNA(AATAT)Rt = 15,30 min Leu-Lys-CNA(AATAT)Rt = 16,08 min Lys-Lys-CNA(AATAT)Rt = 15,34 min Ser-Lys-CNA(AATAT)Rt = 15,29 min |

| | |
|---|---|
| ESI-MS: | Ala-Lys-CNA(AATAT) [M+H]⁺_{calc} 1561,6; [M+H]⁺ₑₓₚ 1561,4 Asp-Lys-CNA(AATAT) [M+H]⁺_{calc} 1605,7; [M+H]⁺ₑₓₚ 1605,3 Leu-Lys-CNA(AATAT) [M+H]⁺_{calc} 1603,8; [M+H]⁺ₑₓₚ 1603,4 Lys-Lys-CNA(AATAT) [M+H]⁺_{calc} 1619,3; [M+H]⁺ₑₓₚ 1619,0 Ser-Lys-CNA(AATAT) [M+H]⁺_{calc} 1577,7; [M+H]⁺ₑₓₚ 1578,7 |

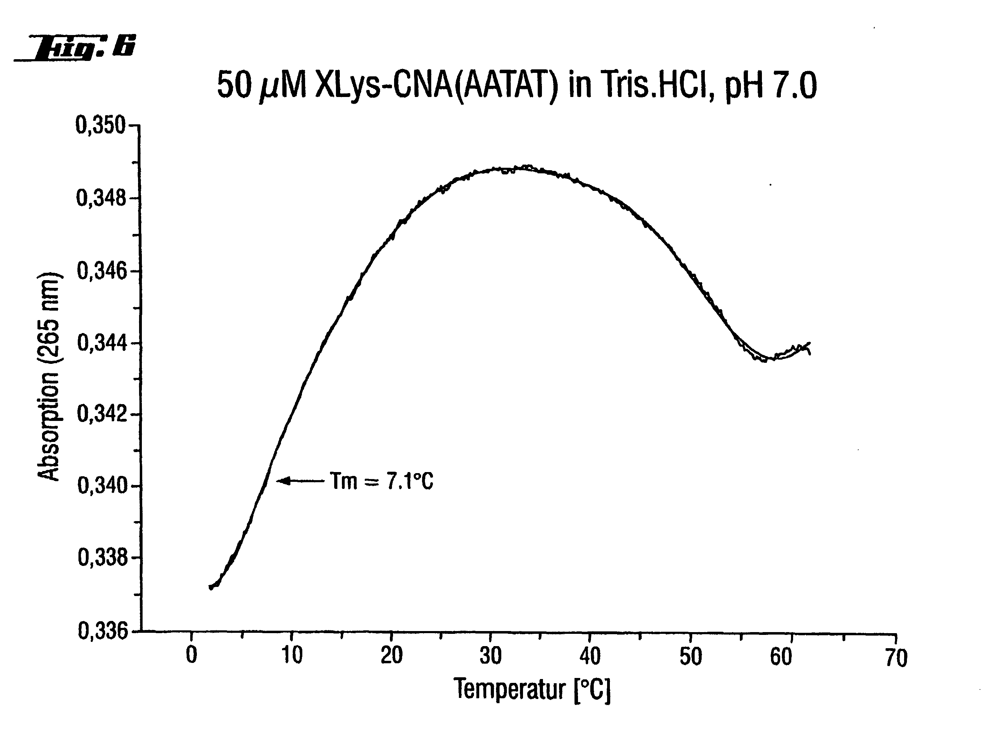

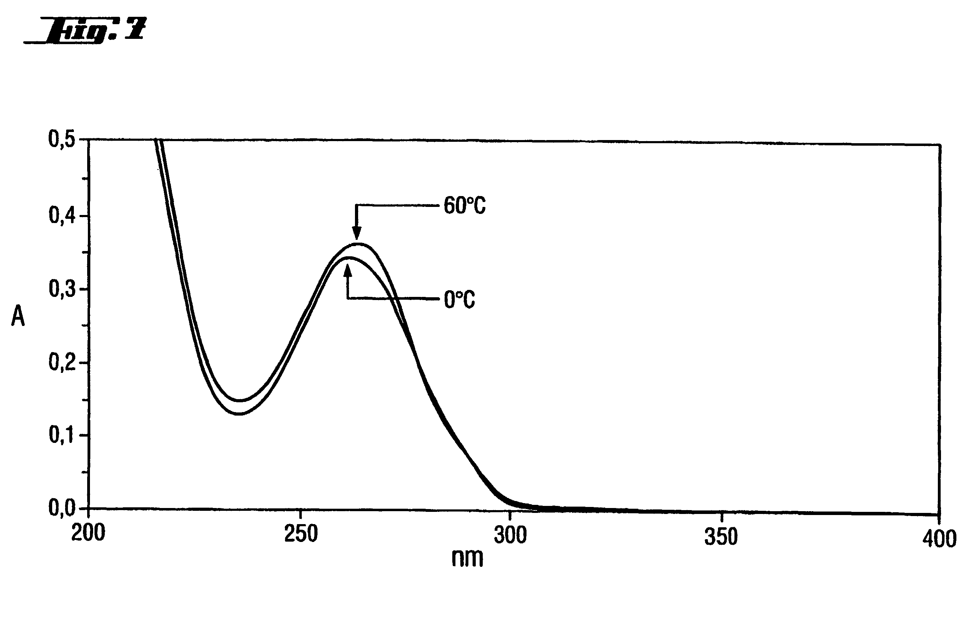

Nach Charakterisierung der Einzelkomponenten wurden die HPLC-Fraktionen vereinigt. Nach Entsalzung der Peptidbibliotheks-CNA-Oligomeren XLys-CNA(AATAT) wurden auf einem Perkin Eimer Lambda 2 UV-VIS Spektrometer die temperaturunabhängigen Extinktionen bei 265 nm bei 50 µM über einen Bereich von 0-60°C gemessen (in TrisHCl Puffer bei pH 7,0). Die erste Ableitung dieser Temperaturkurve ergibt die Schmelztemperatur (Tm = 7°C bei 50 µM) (Fig. 6). Die UV-Spektren von XLys-CNA(AATAT) bei 0°C und 60°C unterscheiden sich in qualitativ gleicher Weise wie das Pentamer ohne Bibliothek CNA(AATAT). Die Wellenlänge des Absorptionsmaximums verschiebt sich von 261,4 nm (E = 0,3427) bei 0°C zu 263,8 nm (E = 0,3626) bei 60°C und belegt damit die Existenz der supramolekularen Komplexe, d.h. einer äquilibrierenden kombinatorischen Bibliothek (Fig. 7.).

### Hierbei bedeuten

- Boc: tert.-Butyloxycarbonyl
- DCM: Dichlormethan
- DIC: Diisopropyl-Carbodiimid
- DIEA: Diisopropylethylamin
- DMAP: Dimethylaminopyridin
- DMF: Dimethylformamid
- Fmoc: Fluorenylmethyloxycarbonyl
- HATU: O-[7-Azabenzotriazol-1-yl]-1,1,3,3-Tetramethyluroniumhexafluorophosphat
- TFA: Trifluoressigsäure

## Patentansprüche

1. Substanzbibliothek, enthaltend ein molekulares Paarungssystem, wobei mindestens zwei der molekularen Spezies, die das Paarungssystem bilden, jeweils an mindestens eine gleiche oder verschiedene molekulare Spezies kovalent gebunden sind und wobei das molekulare Paarungssystem reversibel durch nicht-kovalente Kräfte ausgebildet wird.

2. Substanzbibliothek nach Anspruch 1, **dadurch gekennzeichnet, daß** die molekularen Spezies in einer Substanzbibliothek enthalten sind.

3. Substanzbibliothek nach Anspruch 1 oder 2, **dadurch gekennzeichnet, daß** das Paarungssystem eine Nukleinsäure ist.

4. Substanzbibliothek nach Anspruch 3, **dadurch gekennzeichnet, daß** das Paarungssystem eine DNA ist.

5. Substanzbibliothek nach Anspruch 3, **dadurch gekennzeichnet, daß** das Paarungssystem eine RNA ist.

6. Substanzbibliothek nach Anspruch 3, **dadurch gekennzeichnet, daß** das Paarungssystem eine Pyranosyl-RNA ist.

7. Substanzbibliothek nach Anspruch 1 ode 2, **dadurch gekennzeichnet, daß** das Paarungssystem eine PNA ist.

8. Substanzbibliothek nach Anspruch 1 oder 2, **dadurch gekennzeichnet, daß** das Paarungssystem eine CNA ist.

9. Substanzbibliothek nach einem oder mehreren der Ansprüche 1-8, **dadurch gekennzeichnet, daß** die molekulare Spezies linear konstituierte Moleküle sind.

10. Substanzbibliothek nach Anspruch 9, **dadurch gekennzeichnet, daß** die molekularen Spezies Peptide sind.

11. Substanzbibliothek nach Anspruch 9, **dadurch gekennzeichnet, daß** die molekularen Spezies Peptoide sind.

12. Substanzbibliothek nach einem oder mehreren der Ansprüche 1-9, **dadurch gekennzeichnet, daß** die molekularen Spezies Oligo- oder Polysaccharide sind.

13. Substanzbibliothek nach Anspruch 9, **dadurch gekennzeichnet, daß** die molekularen Spezies Nukleinsäuren oder deren Analoga sind.

14. Substanzbibliothek nach einem oder mehreren der Ansprüche 1-8, **dadurch gekennzeichnet, daß** die molekularen Spezies Monomere sind.

15. Substanzbibliothek nach einem oder mehreren der Ansprüche 3-8, **dadurch gekennzeichnet, daß** das Paarungssystem aus einem längeren und zwei kürzeren Basensträngen besteht, wobei die beiden kürzeren Stränge an unterschiedlichen Stellen komplementär zum längeren Strang, jedoch zueinander nicht komplementär sind und wobei im Falle der Basenpaarung mit dem längeren Strang zwischen den kürzeren Strängen eine Lücke von wenigstens einer Base verbleibt, während im Bereich dieser Lücke entsprechend deren Größe auf dem längeren Strang mindestens eine Base ungepaart bleibt, wobei jeweils diejenigen Basen der beiden kürzeren Stränge, die sich am Anfang bzw. am Ende der Paarungslücke befinden, über einen Linker mit jeweils einer molekularen Spezies gemäß einem oder mehreren der Ansprüche 1, 2 und 9 bis 14 verknüpft sind, während mindestens eine der ungepaarten Basen des längeren Stranges mit einer molekularen Spezies gemäß einem oder mehreren der Ansprüche 1, 2 und 9 bis 14 über einen Linker verknüpft ist.

16. Verfahren zur Herstellung eines supramolekularen Komplexes, **dadurch gekennzeichnet, daß** man eine Substanzbibliothek gemäß einem der Ansprüche 1 bis 15 einer Wechselwirkung mit einem Substrat aussetzt und den dabei gebildeten supramolekularen Komplex identifiziert und gegebenenfalls isoliert.

17. Verfahren nach Anspruch 16, **dadurch gekennzeichnet, daß** das Substrat ein Peptid ist.

18. Verfahren nach Anspruch 16, **dadurch gekennzeichnet, daß** das Substrat ein Peptoid ist.

19. Verfahren nach Anspruch 16, **dadurch gekennzeichnet, daß** das Substrat ein Oligo- oder Polysaccharid ist.

20. Verfahren nach Anspruch 16, **dadurch gekennzeichnet, daß** das Substrat eine Nukleinsäure oder deren Analogon ist.

21. Verfahren nach Anspruch 16, **dadurch gekennzeichnet, daß** das Substrat ein Arzneistoff ist.

22. Verfahren nach Anspruch 16, **dadurch gekennzeichnet, daß** das Substrat ein Pflanzenschutzwirkstoff ist.

23. Verfahren nach Anspruch 16, **dadurch gekennzeichnet, daß** das Substrat ein Analogon eines oder mehrerer Moleküle im Übergangszustand einer chemischen Reaktion ist.

24. Verfahren nach Anspruch 16, **dadurch gekennzeichnet, daß** das Substrat ein Metabolit ist:

25. Verfahren nach Anspruch 16, **dadurch gekennzeichnet, daß** das Substrat ein physiologischer Botenstoff ist.

26. Verfahren nach Anspruch 16, **dadurch gekennzeichnet, daß** das Substrat eine Substanz ist, welche im menschlichen oder tierischen Körper im Fall von krankhaften Veränderungen produziert oder im erhöhten Maße produziert wird.

27. Verfahren nach Anspruch 16, **dadurch gekennzeichnet, daß** das Substrat Angriffspunkte von Pharmaka, bevorzugt Rezeptoren, spannungsabhängige Ionenkanäle, Transporter, Enzyme und Biosynthese-Einheiten von Mikroorganismen ist.

28. Supramolekularer Komplex, enthaltend mindestens einen Vertreter der Substanzbibliothek gemäß der Anspräche 1 bis 15 und ein Substrat.

29. Verwendung eines supramolekularen Komplexes gemäß Anspruch 28 zur Herstellung eines Arzneiwirkstoffes.

30. Verwendung eines supramolekularen Komplexes gemäß Anspruch 28 zur Herstellung eines Pflanzenschutzwirkstoffes.

31. Verwendung eines supramolekularen Komplexes gemäß Anspruch 28 zur Herstellung eines Katalysators.

32. Verwendung eines supramolekularen Komplexes gemäß Anspruch 28 zur in-vitro Diagnose von Krankheiten.

33. Verwendung eines supramolekularen Komplexes gemäß Anspruch 28 zur Herstellung eines Diagnose-Kits.

34. Diagnose-Kit enthaltend einen supramolekularen Komplex gemäß Anspruch 28.

35. Verwendung einer Substanzbibliothek gemäß einem oder mehreren der Ansprüche 1 bis 15 zur in-vitro Diagnose von Krankheiten.

36. Verwendung einer Substanzbibliothek gemäß einem oder mehreren der Ansprüche 1 bis 15 zur Herstellung eines Diagnose-Kits.

37. Verwendung einer Substanzbibliothek gemäß einem oder mehreren der Ansprüche 1 bis 15 zur Herstellung eines Katalysators.

38. Verfahren zur Herstellung einer Substanzbibliothek gemäß einem oder mehreren der Ansprüche 1 bis 15, **dadurch gekennzeichnet, daß** man molekulare Spezies, die verschieden oder gleich sein können, an ein Paarungssystem koppelt.

39. Cyclohexylnucleooligo-Amid enthaltend Aminocyclohexylethansäure-Einheiten.

## Claims

1. Substance library comprising a molecular pairing system, where at lest two of the molecular species forming the pariring system are each covalently bonded to at least one identical or different molecular species, and where the molecular pairing system is formed reversibly through non-covalent forces.

2. Substance library according to Claim 1, **characterized in that** the molecular species are present in a substance library.

3. Substance library according to Claim 1 or 2, **characterized in that** the pairing system is a nucleic acid.

4. Substance library according to Claim 3, **characterized in that** the pairing system is a DNA.

5. Substance library according to Claim 3, **characterized in that** the pairing system is an RNA.

6. Substance library according to Claim 3, **characterized in that** the pairing system is a pyranosyl-RNA.

7. Substance library according to Claim 1 or 2, **characterized in that** the pairing system is a PNA.

8. Substance library according to Claim 1 or 2, **characterized in that** the pairing system is a CNA.

9. Substance library according to one or more of Claims 1 to 8, **characterized in that** the molecular species are molecules with a linear constitution.

10. Substance library according to Claim 9, **characterized in that** the molecular species are peptides.

11. Substance library according to Claim 9, characterzed in that the molecular species are peptoids.

12. Substance library according to one or more of Claims 1 to 9, **characterized in that** the molecular species are oligo- or polysaccharides.

13. Substance library according to Claim 9, **characterized in that** the molecular species are nucleic acids or analogues thereof.

14. Substance library according to one or more of Claims 1 to 8, **characterized in that** the molecular species are monomers.

15. Substance library according to one or more of Claims 3 to 8, **characterized in that** the pairing system consists of one longer and two shorter base strands, with the two shorter strands being complementary to the longer strand at different points but not being complementary to one another, and with a gap of at least one base remaining between the short strands in the event of base-pairing with the longer strand, while at least one base remains unpaired in the region of this gap corresponding to the size thereof on the longer strand, with those bases on each of the two shorter strands which are located at the start and at the end of the pairing gap being linked by a linker to one molecular species according to one or more of Claims 1, 2 and 9 to 14 in each case, while at least one of the unpaired bases in the longer strand is linked by a linker to a molecular species according to one or more of Claims 1, 2 and 9 to 14.

16. Process for the preparation of a supramolecular complex, **characterized in that** it comprises exposing a substance library according to any of Claims 1 to 15 to an interaction with a substrate, and identifying and, where appropriate, isolating the supramolecular complex formed thereby.

17. Process according to Claim 16, **characterized in that** the substrate is a peptide.

18. Process according to Claim 16, **characterized in that** the substrate is a peptoid.

19. Process according to Claim 16, **characterized in that** the substrate is an oligo- or polysaccharide.

20. Process according to Claim 16, **characterized in that** the substrate is a nucleic acid or analogue thereof.

21. Process according to Claim 16, **characterized in that** the substrate is a medicinal substance.

22. Process according to Claim 16, **characterized in that** the substrate is an active substance for crop protection.

23. Process according to Claim 16, **characterized in that** the substrate is an analogue of one or more molecules in the transition state of a chemical reaction.

24. Process according to Claim 16, **characterized in that** the substrate is a metabolite.

25. Process according to Claim 16, **characterized in that** the substrate is a physiological messenger.

26. Process according to Claim 16, **characterized in that** the substrate is a substance which is produced, or produced to an increased extent, in the human or animal body in the event of pathological changes.

27. Process according to Claim 16, **characterized in that** the substrate is sites of action of drugs, preferably receptors, voltage-dependent ion channels, transporters, enzymes and biosynthetic units of microorganisms.

28. Supramolecular complex comprising at least one representative of the substance library according to Claims 1 to 15 and a substrate.

29. Use of a supramolecular complex according to Claim 28 for the preparation of a medicinal active substance.

30. Use of a supramolecular complex according to Claim 28 for the preparation of an active substance for crop protection.

31. Use of a supramolecular complex according to Claim 28 for the preparation of a catalyst.

32. Use of a supramolecular complex according to Claim 28 for the in-vitro diagnosis of diseases.

33. Use of a supramolecular complex according to Claim 28 for the production of a diagnostic kit.

34. Diagnostic kit comprising a supramolecular complex according to Claim 28.

35. Use of a substance library according to one or more of Claims 1 to 15 for the in-vitro diagnosis of diseases.

36. Use of a substance library according to one or more of Claims 1 to 15 for the production of a diagnostic kit.

37. Use of a substance library according to one or more of Claims 1 to 15 for the preparation of a catalyst.

38. Process for the preparation of a substance library according to one or more of Claims 1 to 15, which comprises coupling molecular species, which may be different or identical, to a pairing system.

39. Cyclohexylnucleooligoamide comprising aminocyclohexylethanoic acid units.

## Revendications

1. Bibliothèque de substances, contenant un système d'appariement moléculaire, dans laquelle au moins deux des espèces moléculaires, qui forment le système d'appariement, sont respectivement liées par covalence à au moins une espèce moléculaire identique ou différente et dans laquelle le système d'appariement moléculaire est formé de manière réversible par des forces non covalentes.

2. Bibliothèque de substances selon la revendication 1, **caractérisée en ce que** les espèces moléculaires sont contenues dans une bibliothèque de substances.

3. Bibliothèque de substances selon la revendication 1 ou 2, **caractérisée en ce que** le système d'appariement est un acide nucléique.

4. Bibliothèque de substances selon la revendication 3, **caractérisée en ce que** le système d'appariement est un ADN.

5. Bibliothèque de substances selon la revendication 3, **caractérisée en ce que** le système d'appariement est un ARN.

6. Bibliothèque de substances selon la revendication 3, **caractérisée en ce que** le système d'appariement est un ARN-pyranosyle.

7. Bibliothèque de substances selon la revendication 1 ou 2, **caractérisée en ce que** le système d'àppariement est un composé PNA.

8. Bibliothèque de substances selon la revendication 1 ou 2, **caractérisée en ce que** le système d'appariement est un composé CNA.

9. Bibliothèque de substances selon une ou plusieurs des revendications 1 à 8, **caractérisée en ce que** les espèces moléculaires sont des molécules de constitution linéaire.

10. Bibliothèque de substances selon la revendication 9, **caractérisée en ce que** les espèces moléculaires sont des peptides.

11. Bibliothèque de substances selon la revendication 9, **caractérisée en ce que** les espèces moléculaires sont des peptoides.

12. Bibliothèque de substances selon une ou plusieurs des revendications 1 à 9, **caractérisée en ce que** les espèces moléculaires sont dès oligo- ou polysaccharides.

13. Bibliothèque de substances selon la revendication 9, **caractérisée en ce que** les espèces moléculaires sont des acides nucléiques ou leurs analogues.

14. Bibliothèque de substances selon une ou plusieurs des revendications 1 à 8, **caractérisée en ce que** les espèces moléculaires sont des monomères.

15. Bibliothèque de substances selon une ou plusieurs des revendications 3 à 8, **caractérisée en ce que** le système d'appariement est constitué d'un brin de base plus long et de deux plus courts, dans laquelle les deux brins plus courts sont complémentaires à des endroits différents du brin plus long, mais ne sont pas complémentaires entre eux et dans laquelle dans le cas de l'appariement des bases avec le brin plus long il reste entre les brins plus courts une lacune d'au moins une base, tandis que dans la zone de cette lacune il reste sur le brin plus long au moins une base non appariée dont la taille correspond à cette lacune, dans laquelle respectivement les bases des deux brins plus courts qui se trouvent au début ou la fin de la lacune d'appariement, sont reliées par un chaînon avec respectivement une espèce moléculaire selon une ou plusieurs des revendications 1, 2 et 9 à 14, tandis qu'au moins une des bases non appariées du brin plus long est reliée par un chaînon avec une espèce moléculaire selon une ou plusieurs des revendications 1, 2 et 9 à 14.

16. Procédé pour la préparation d'un complexe supramoléculaire, **caractérisé en ce qu'**on expose une bibliothèque de substances selon une des revendications 1 à 15 à un effet d'échange avec un substrat et on identifie le complexe supramoléculaire ainsi formé et on l'isole éventuellement.

17. Procédé selon la revendication 16, **caractérisé en ce que** le substrat est un peptide.

18. Procédé selon la revendication 16, **caractérisé en ce que** le substrat est un peptoide.

19. Procédé selon la revendication 16, **caractérisé en ce que** le substrat est un oligo- ou polysaccharide.

20. Procédé selon la revendication 16, **caractérisé en ce que** le substrat est un acide nucléique ou son analogue.

21. Procédé selon la revendication 16, **caractérisé en ce que** le substrat est un médicament.

22. Procédé selon la revendication 16, **caractérisé en ce que** le substrat est une substance active phytosanitaire.

23. Procédé selon la revendication 16, **caractérisé en ce que** le substrat est un analogue d'une ou de plusieurs molécules dans l'état de transition d'une réaction chimique.

24. Procédé selon la revendication 16, **caractérisé en ce que** le substrat est un métabolite.

25. Procédé selon la revendication 16, **caractérisé en ce que** le substrat est un messager physiologique.

26. Procédé selon la revendication 16, **caractérisé en ce que** le substrat est une substance qui est produite ou est produite en quantité augmentée dans les corps humains et animaux dans le cas d'altérations maladives.

27. Procédé selon la revendication 16, **caractérisé en ce que** le substrat représente des points d'attaque de médicaments, de préférence des récepteurs, des canaux ioniques dépendant du potentiel, des transporteurs, des enzymes et des unités de biosynthèse de micro-organismes.

28. Complexe supramoléculaire, contenant au moins un représentant de la bibliothèque de substances selon les revendications 1 à 15 et un substrat.

29. Utilisation d'un complexe supramoléculaire selon la revendication 28 pour la préparation d'une substance médicamenteuse.

30. Utilisation d'un complexe supramoléculaire selon la revendication 28 pour la préparation d'une substance active phytosanitaire.

31. Utilisation d'un complexe supramoléculaire selon la revendication 28 pour la préparation d'un catalyseur.

32. Utilisation d'un complexe supramoléculaire selon la revendication 28 pour le diagnostic in vitro de maladies.

33. Utilisation d'un complexe supramoléculaire selon la revendication 28 pour la préparation d'un ensemble de diagnostic.

34. Ensemble de diagnostic contenant un complexe supramoléculaire selon la revendication 28.

35. Utilisation d'une bibliothèque de substances selon une ou plusieurs des revendications 1 à 15 pour le diagnostic in vitro de maladies.

36. Utilisation d'une bibliothèque de substances selon une ou plusieurs des revendications 1 à 15 pour la préparation d'un ensemble de diagnostic.

37. Utilisation d'une bibliothèque de substances selon une ou plusieurs des revendications 1 à 15 pour la préparation d'un catalyseur.

38. Procédé pour la préparation d'une bibliothèque de substances selon une ou plusieurs des revendications 1 à 15, **caractérisé en ce qu'**on couple à un système d'appariement des espèces moléculaires, qui peuvent être identiques ou différentes.

39. Cyclohexylnucléooligo-amide contenant des motifs d'acide aminocyclohexyléthanoïque.
